# EUROPÄISCHE PATENTSCHRIFT

(11) **EP 0 831 674 B1**
(45) Veröffentlichungstag und Bekanntmachung des Hinweises auf die Patenterteilung: **14.11.2001**
(21) Anmeldenummer: 97114604.8
(22) Anmeldetag: 22.08.1997
(51) Int. Cl.: H04R 25/00

(54) **Vollständig implantierbare Hörhilfe mit elektrischer Reizung des Gehörs**
Fully implantable hearing aid with electrical stimulation of auditory system
Prothèse auditive entièrement implantable avec stimulation électrique de l'ouie

(30) Priorität: 18.09.1996 DE 19638159
(43) Veröffentlichungstag der Anmeldung: 25.03.1998
(73) Patentinhaber: IMPLEX Aktiengesellschaft Hearing Technology, 85737 Ismaning (DE)
(72) Erfinder: Leysieffer, Hans, Dr. Dipl.-Ing., 82024 Taufkirchen (DE)
(74) Vertreter: Schwan, Gerhard, Dipl.-Ing.

(56) Entgegenhaltungen:
- EP-A- 0 340 594
- EP-A- 0 587 032
- DE-A- 3 918 329
- DE-U- 29 608 215
- US-A- 4 217 403
- US-A- 4 918 745
- US-A- 5 024 224
- PATENT ABSTRACTS OF JAPAN vol. 1995, no. 4, 31. Mai 1995 (1995-05-31) & JP 07 023493 A (AUDIO TECHNICA), 24. Januar 1995 (1995-01-24)
- PATENT ABSTRACTS OF JAPAN vol. 14, no. 79 (E-0888), 14. Februar 1990 (1990-02-14) & JP 01 291600 A (UBE IND LTD), 24. November 1989 (1989-11-24)

## Beschreibung

Elektronische Hörprothesen, die mit direkter elektrischer Stimulation über Reizelektroden das Gehör anregen, haben heute einen Entwicklungsstand erreicht, der die routinemäßige Implantation bei solchen Patienten erlaubt, deren Gehör ganz oder nahezu vollständig durch Unfall, Krankheit oder sonstige Einflüsse ausgefallen oder bereits von Geburt an nicht funktionsfähig ist. Ist in diesen Fällen nur das Innenohr (Cochlea) und nicht die nach zentral führende neuronale Hörbahn betroffen, kann mit elektrischen Reizsignalen der verbliebene Hömerv stimuliert und somit ein Höreindruck erzeugt werden, der bei entsprechender elektronischer Sprachsignalvorverarbeitung bis zu einem offenen Sprachverständnis führen kann. Diese Systeme werden nach heute üblichem Sprachgebrauch als Cochlea Implantate bezeichnet.

Bei diesen Cochlea Implantaten wird ein ein- oder mehrkanaliges Reizelektroden-Array in die Cochlea eingeführt, das von einem elektronischen System angesteuert wird, wobei dieses hermetisch dichte und biokompatibel eingekapselte Elektronikmodul operativ im knöchemen Bereich hinter dem Ohr (Mastoid) eingebettet ist. Das elektronische System enthält jedoch im wesentlichen nur Dekodier- und Treiberschaltungen für die Reizelektroden; die akustische Schallaufnahme, die Wandlung dieses Schallsignals in elektrische Signale und deren weitere Verarbeitung erfolgen grundsätzlich extern in einem sogenannten Sprachprozessor, der außen am Körper getragen wird. Der Sprachprozessor setzt die vorverarbeiteten Signale entsprechend meist digital kodiert auf ein hochfrequentes Trägersignal um, das über eine induktive Kopplung durch die geschlossene Haut (transkutan) zu dem Implantat übertragen wird. Das schallaufnehmende Mikrofon befindet sich ausnahmslos außerhalb des Körpers und in den meisten Anwendungen in einem an der Ohrmuschel getragenen Gehäuse eines Hinter-dem-Ohr-Hörgerätes (HdO) und ist mit einem Kabel mit dem Sprachprozessor verbunden. Der Sprachprozessor erhält seine Betriebsenergie aus eingebauten Primärbatterien oder wiederaufladbaren Akkumulatoren. Das Implantat selbst enthält keinen elektrisch langzeitwirksamen Energiespeicher, sondern bezieht seine Betriebsenergie aus dem hochfrequenten, transkutan und induktiv eingekoppelten Trägersignal des Sprachprozessors.

Aus den oben genannten Gründen sind alle heute bekannten und verfügbaren Cochlea Implantat-Systeme teilimplantierbar, das heißt, daß das Gesamtsystem nur durch das Zusammenspiel externer und implantierbarer Komponenten betriebsbereit ist.

Die wissenschaftlichen Grundlagen und klinischen Applikationen der Cochlea Implantate sind in zahlreichen Publikationen dargestellt; ein Überblick des jeweilig aktuellen Standes der Wissenschaft und Technik ist beispielhaft gegeben in: Merzenich, M. M. et al.: "Electrical Stimulation Of The Acoustic Nerve In Man", Velo-Bind, San Francisco, 1974; Schindler, R. A. und Merzenich, M. M. (eds): "Cochlear Implants", Raven Press, New York, 1985; Hoke, M. (ed): "Cochlear Implants in Clinical Use", Advances in Audiology, Vol. 2, Karger Basel, 1984; Miller, J. M. und Spelman, F. A. (eds): "Cochlear Implants", Springer New York, 1989; Hochmair-Desoyer, I. J. und Hochmair, E.S. (eds): "Advances in Cochlear Implants", MANZ Wien, 1993. Die technischen Realisierungsvarianten der Implantatkomponenten und extrakorporalen Teile der Cochlea Implantate wie Mikrofone, deren elektrische Speisung, Sprachprozessorausführungsformen, Sprachkodierungsstrategien und transkutane Energie- und Datenübertragungsprinzipien zum implantierten Systemteil sind in zahlreichen Patentveröffentlichungen dokumentiert: EP-A-0 572 382 (Daly), WO-A-92/22107 (Kuzma), AU 624989 (Seligman und Dowell), US-A-4 532 930 (Crosby et al.), EP-A-0 076 070 (Hochmair), AU 314490 (Dooley et al.), AU 6776784 (Harrison und Seligman), WO-A-89/06988 (Kuzma), DE-A-34 20 244 (Hortmann und Kunick), und AU 619689 (Seligman).

Der grundsätzliche Nachteil aller oben genannten Cochlea Implantat-Systeme, der insbesondere bei der heute anerkannt wirksamen und sicheren Versorgung bei Kindern zum Tragen kommt, besteht darin, daß zum Betrieb des Implantates der außen am Körper zu tragende Sprachprozessor, das externe Mikrofon, die Sendespule und die zugehörigen Kabelverbindungen grundsätzlich notwendig sind. Dies bedeutet eine Behinderung im alltäglichen Leben sowie eine Stigmatisierung des Patienten durch die sichtbaren äußeren Komponenten der Hörprothese.

Diese gravierenden Nachteile sind vollständig nur dadurch zu umgehen, daß das Cochlea Implantat mit allen technisch notwendigen Betriebskomponenten gänzlich implantiert wird. Dies erfordert folgende technische und klinische Lösungen:
A) Das elektronische Signalverarbeitungssystem muß in das Implantat integriert werden.
B) Die Energieversorgung des Gesamtsystems muß implantierbar ausgeführt sein.
C) Das schallaufnehmende Mikrofon ist so zu konzipieren, daß es ebenfalls vollständig unter der geschlossenen Körperoberfläche implantiert werden kann.

### Zu A):

Die moderne Mikroelektronik stellt heute Miniaturisierungsformen zur Verfügung, welche die vollständige Integration aller notwendigen Signalverarbeitungskomponenten bei minimierter elektrischer Leistungsaufnahme ermöglichen. Dies gilt insbesondere für vollständig implantierte Geräte, weil in diesem Fall die technisch und energetisch aufwendige, transkutane Hochfrequenz-Übertragungsstrecke für Daten und Betriebsenergie nicht notwendig ist.

### Zu B):

Eine Möglichkeit des energetischen Betriebes eines aktiven Implantates besteht in der Integration einer Primärbatterie. Dies ist aus dem heutigen Stand der Technik der Herzschrittmacher bekannt Bei höherem Bedarf an kontinuierlicher Betriebsenergie wie im Fall einer aktiven Hörhilfe kann ein elektrischer Akkumulator in das Implantat integriert werden, der nur im Bedarfsfall nach Entladung von außen transkutan und auf induktivem Weg nachgeladen wird. Solche Energieversorgungssysteme sind beispielhaft für Herzschrittmacher dargestellt worden (DE-A-19 40 803, US-A-4 134 408). Im Falle implantierbarer Hörprothesen wurden solche nachladbaren Akkumulatorsysteme insbesondere für Hörgeräte mit elektromechanischer Stimulation des geschädigten Innenohres angegeben (DE-A-41 04 359, US-A-5 279 292, EP-A-0 499 939, DE-A-39 18 086, EP-A-0 400 630, DE-A-39 18 086 und US-A-5 411 467).

### Zu C):

Prinzipielle Lösungsmöglichkeiten eines implantierbaren Mikrofons für Hörhilfen sind ebenfalls für Hörgeräte mit elektromechanischer Stimulation dargestellt worden:

Schaefer (US-A-4 729 366, US-A-4 850 962 und EP-A-0 263 254) beschreibt ein implantierbares Hörgerät, bei dem nach Entfernen des Amboß der Ossikelkette ein zweifaches Wandlersystem eingesetzt wird, wobei ein Element als mechano-elektrischer Wandler (Sensor: Mikrofon-Funktion) und das andere Element als elektromechanischer Wandler zur vibratorischen Stimulation des Steigbügels (Aktor-Funktion) dienen. Das Sensorelement ist mit dem verbleibenden Hammer über ein Koppelelement, das als mechanisch steifer Draht ausgeführt ist, verbunden und nimmt so die mechanischen Schwingungen des Trommelfells auf, die aus einer auf das Trommelfell einfallenden akustischen Welle resultieren. Bei der Sensorankopplung sind verschiedene Varianten des Koppelelementes an den Hammergriff, den Hammerkopf und den Körper des Hammers dargestellt Problematisch erscheinen bei der beschriebenen Mikrofonfunktions-Realisierung folgende Aspekte:
- Bei Verwendung eines piezokeramischen Wandlerelementes, das beispielhaft als stabförmiger Bimorph-Biegeschwinger ausgeführt ist, liegt die mechanische Eingangsimpedanz deutlich über der biologischen mechanischen Quellimpedanz, die in diesem Fall hauptsächlich von dem System Trommelfell mit dem damit fest verwachsenen Hammer gebildet wird. Daraus resuliert bei gegebenem Eingangsschalldruckpegel im äußeren Gehörgang eine sehr kleine Auslenkung des Piezoelementes, was wiederum bei gegebenem mechano-elektrischen Wandlungsfaktor des Piezoelementes zu sehr kleinen elektrischen Ausgangssignalen führt. Die daher notwendige hohe elektrische Verstärkung in dem signalverarbeitenden Modul reduziert bei heute gegebener, mikroelektronischer Verstärkertechnologie die nutzbare Dynamik des Gesamtsystem aufgrund eines unzureichenden Signal-Stör-Abstandes.
- Auch wenn oben genannter Nachteil durch Verwendung eines Wandlersystems mit deutlich reduziertem mechanischem Impedanzniveau wie z.B. dünne Piezofilme (z.B. PFDV, Polyvinylidenfluorid) abgeschwächt wird, bleibt ein weiterer Nachteil dieser Variante einer Mikrofon-Funktion bestehen: aufgrund heute bekannter komplexer Resonanzeigenschaften des Mittelohrapparates einschließlich des Trommelfells (z.B. "Analysis of dynamic behavior of human middle ear using a finite-element method", Wada, H. et al., J. Acoust. Soc. Am. 92 (6), Dec. 1992, pp. 3157 - 3168) kann nicht davon ausgegangen werden, daß bei angenommenem frequenzunabhängigem Eingangsschalldruck ein ebenfalls über der Frequenz ebener Schalldruckübertragungsfaktor erzielt wird. Daraus resultieren lineare Verzerrungen der Mikrofon-Übertragungsfunktion, die für die angestrebte Hörgeräte-Funktion unerwünscht und darüberhinaus inter-individuellen Schwankungen aufgrund unterschiedlicher Anatomie unterworfen sind.
- Die nicht näher ausgeführte technische Realisierung der notwendigen hermetisch dichten und biokompatiblen Kapselung des Sensorelementes erscheint insbesondere auch deshalb sehr schwierig, weil die mechanischen Schwingungen des Koppeldrahtes, der den Hammer mit dem Wandlerelement verbindet, durch die zwingend hermetisch dichte Wandung des Implantatgehäuses, welches das aktive Wandlerelement enthält, geleitet werden müssen.

Engebretson et al. (US-A-4 988 333) beschreiben ein implantierbares akustisches Kuppler-System, bei dem eine mit einer beispielhaft aus Silikon bestehenden dünnen, kreisförmigen Membran abgeschlossene Kammer als schallaufnehmendes Element ausgebildet ist. Beispielhaft kann diese Membran über einen dünnen Koppeldraht, der im Mittelpunkt der Membran befestigt ist, mechanisch fest mit dem Hammer der Ossikelkette verbunden werden, wodurch die Trommelfellschwingungen als akustisches Signal innerhalb der Kammer abgebildet werden. Über einen Schalleitungsschlauch, der an diese aufnehmende Kuppelkammer angeschlossen ist, wird das Schallsignal zu einem Mikrofon geleitet, das aus Platzgründen nicht in der Paukenhöhle untergebracht ist, sondern in einem Implantat-Hauptgehäuse im Bereich des Antrums oder Mastoids. Dieses System wurde in einer miniaturisierten Ausführungsform (4,0 mm Durchmesser der Kuppelkammer) in seiner prinzipiellen Funktion in einem zweiwöchigen Tiermodell getestet (Deddens et al.: "Totally implantable hearing aids: the effects of skin thickness on microphone function", Am. J. Otolaryngol. 1990, 11, pp. 1-4), wobei die akustische Kuppelkammer mit einer 100 mm dicken Silikonscheibe als Schalleintrittsmembran abgeschlossen war.

Das grundlegende Prinzip einer Schallweiterleitung über ein Schlauchelement wird von weiteren Autoren beschrieben (Mahoney, US-A-3 346 704 und US-A-3 557 775, Nunley et al., US-A-3 882 285, Leysieffer et al., DE-39 40 632). Das eigentliche Mikrofonelement ist in allen beschriebenen Fällen in einem gekapselten Implantatgehäuse plaziert. Die Schalleinleitung zu diesem Mikrofon erfolgt über einen mechanisch starren oder flexiblen Schlauch, dessen Eingangsende beispielhaft mit einer dünnen Membran abgeschlossen ist, über die das einfallende Schallsignal aufgenommen wird. Der Ort der Schallaufnahme ist bei Mahoney der Bereich des Mastoids hinter dem Außenohr (Schlauchende mit Membran unter der geschlossenen Haut über dem Mastoid), bei Nunley et al. der Bereich des äußeren Gehörgangs, wobei der Zuleitungsschlauch von der dünnen Haut der Gehörgangswand bedeckt ist, und bei Leysieffer et al. die Paukenhöhle selbst, in die das mit einer dünnen Membran abgeschlossene Ende des Schallzuleitungsschlauches ragt.

Bei diesen Lösungen erscheinen folgende Aspekte als problematisch bzw. technisch schwierig zu realisieren:
- Neben dem eigentlichen Mikrofon sind zusätzliche volumenbehaftete Elemente notwendig, die die Implantierbarkeit bei den ohnehin sehr begrenzten Platzverhältnissen im Bereich des Mittelohres, Antrums und Mastoids erschweren und die unter Langzeitaspekten zu fordernde Biostabilität fraglich erscheinen lassen.
- Technisch ist unter heute allgemein bekannten Aspekten der Wasserdampfaufnahme von Silikonen im Volumenprozentbereich die Membran, die die Schlauchelemente oder Kuppelkammern schalleingangsseitig abschließt, die für die Langzeitstabilität zu fordernde hermetische Dichtheit des Implantates nicht zu realisieren, d.h., es besteht das wesentliche Risiko, daß einerseits die elektronischen Implantatkomponenten wie insbesondere das Mikrofon selbst durch eintretende Feuchtigkeit zerstört bzw. geschädigt werden und andererseits toxische Stoffe aus dem Implantat austreten und in das Körpergewebe eindringen können.
- Funktional bestehen ähnliche Einschränkungen des individuell reproduzierbaren, frequenzunabhängigen Übertragungsmaßes dieser Mikrofonsysteme wie bei dem System nach Schäfer (s.o.), da die dünnen Membranen und schalleitenden Schläuche lineare Verzerrungen bedingen können, die im Einzelfall mit aufwendigen elektronischen Maßnahmen kompensiert werden müssen.

Eine andere Variante wird von Suzuki und Yanigahara angegeben ("Middle Ear Implants: Implantable Hearing Aids", Hoke, M. (ed), Advances in Audiology, Vol. 4, Karger Basel, 1988). Ein zylinderförmiges Edelstahlgehäuse ist auf einer Seite mit einer kreisförmigen dünnen Edelstahlmembran hermetisch dicht abgeschlossen. Innerhalb dieses Gehäuses ist ein konventionelles Miniatur-Elektret-Mikrofon untergebracht, dessen Schalleintrittsöffnung in einen ebenfalls dichten Zwischenraum ragt, der von der Gehäusemembran und einer internen Zwischenwandung gebildet wird. Die drei elektrischen Mikrofonanschlüsse werden über eine dreipolige hermetische Signaldurchführung, die neben der Mikrofonkapsel plaziert ist, aus dem Edelstahlgehäuse herausgeführt und sind mit der sich anschließenden implantierbaren Mikrofonanschlußleitung verbunden. Das Gehäuseinnere ist aus Gründen des Korrosionsschutzes mit einem Edelgas (Argon) gefüllt Das Gehäuse wird in der Mastoidhöhle so implantiert, daß die Gehäuseseite mit der kreisförmigen Membran in einer entsprechenden Bohrung in der hinteren Gehörgangswand des knöchernen Bereich des Gehörgangs plaziert wird und die Gehörgangshaut durch vollständige Bedeckung in direktem mechanischem Kontakt zu der dünnen Edelstahlmembran steht. Fällt eine Schallwelle in den Gehörgang, wird die Metallmembran mit der darüberliegenden Gehörgangshaut in mechanische Schwingungen versetzt, was zu einer Druckschwankung in dem kleinvolumigen Zwischenraum im Inneren des Gehäuses führt. Diese Druckschwankung wird von dem internen Mikrofon als entsprechendes akustisches Signal aufgenommen und in ein elektrisches Signal umgewandelt, das über die Anschlußleitung der weiterverarbeitenden Elektronik zugeführt werden kann. Hierbei ist das System so ausgelegt, daß die erste mechanische Resonanz der schwingfähigen Metallmembran spektral am oberen Ende des audiologischen Übertragungsbereiches liegt und das Schalldruckübertragungsmaß unterhalb dieser Resonanz frequenzunabhängig ist und somit keine linearen Verzerrungen auftreten. Die Gehörgangshaut stellt im wesentlichen einen additiven dynamischen Massenbelag für die schwingfähige Membran dar, so daß sich die Resonanzfrequenz bei Belag mit der Haut gegenüber dem Leerlauf ohne Hautbelag lediglich zu tieferen Frequenzen verschiebt, ohne daß sich das Übertragungsmaß und damit die Gesamtmikrofonempfindlichkeit verändert.

Das Design ist so ausgelegt, daß die Resonanzfrequenz im implantierten Zustand, d.h. bei Belag mit der Gehörgangshaut, bei 3 bis 5 kHz liegt und somit zumindest grundsätzlich der audiologisch wichtige Frequenzbereich übertragen werden kann. Als Schalldruckübertragungsfaktor wird ca. 2 mV/Pa angegeben. Dieser Wert wird als ausreichend für eine elektronische Weiterverarbeitung mit akzeptablem Signal-Störabstand bezeichnet. Die Außenabmessungen dieses zylinderförmigen Mikrofonmoduls betragen 8,0 mm im Durchmesser und 4,0 mm in der Höhe.

Dieses System weist gegenüber den anderen hier angeführten Realisierungsformen eines implantierbaren Mikrofons folgende Vorteile auf:
- Aufgrund der ganzmetallischen Ausführung ist das Modul hermetisch dicht und somit geschützt gegen Körperflüssigkeiten.
- Die funktionale Gestaltung der schallaufnehmenden Membran ermöglicht einen weitgehend frequenzunabhängigen Schalldruckübertragungsfaktor bis zur ersten Resonanzfrequenz.
- Die Fehlanpassung des mechanischen Systems der Membran gegenüber der akustischen Impedanz der eintreffenden Schallwelle bleibt in einem akzeptablen Bereich, so daß ein technisch verwertbarer Übertragungsfaktor resultiert.

Dieses Modul weist jedoch aufgrund seiner äußeren Geometrie einen wesentlichen Nachteil auf: umfangreiche eigene klinische Ermittlungen haben ergeben, daß es unmöglich ist, ein zylinderförmiges Modul mit einem Gesamtdurchmesser von 8,0 mm so im knöchernen Bereich des Gehörgangs und somit in einer Bohrung in der Gehörgangswand zu plazieren, daß einerseits eine vollständige Bedeckung der kreisförmigen, planen Oberfläche mit Gehörgangshaut erfolgt und andererseits gesichert werden kann, daß dieses Modul unter Langzeitaspekten biostabil implantierbar ist. Dies liegt im wesentlichen daran, daß erstens der äußere Gehörgang einen Durchmesser von unter 10 mm aufweist und so eine kreisförmige Membran mit 8,0 mm Durchmesser nicht als berührende, tangentiale Fläche bezeichnet werden kann, sondern eher eine Schnittebene darstellt, und zweitens die Entnahme eines so großen knöchernen Bereiches in der Gehörgangswand die ausreichende Versorgung der über der Metallmembran verbleibenden Gehörgangshaut als äußerst zweifelhaft erscheinen läßt Insgesamt erscheint es als sehr fraglich, ob die beschriebenen Mikrofonsysteme überhaupt im knöchernen Bereich des Gehörgangs implantierbar sind oder ob eine Plazierung lediglich im knorpeligen oder Weichteile-Bereich des Gehörgangs möglich ist Im letzteren Fall besteht bekannterweise das hohe Risiko des Wandems eines Implantates, da eine adäquate mechanisch feste und langzeitstabile Verankerung des Mikrofongehäuses in knöchernen Strukturen nicht möglich ist.

Des weiteren ist aus der EP-A-0 340 594 ein vollständig implantierbares Hörhilfegerät bekannt, bei welchem in einem Gehäuse eine Batterie und ein über eine externe drahtlose Fernbedienung ansteuerbarer Verstärkerteil untergebracht sind. Von einem in einem separaten Mikrofongehäuse untergebrachten Mikrofon ankommende Signale werden mittels des Verstärkerteils verstärkt und an einen piezoelektrischen Vibrator zur Anregung des Gehörs geleitet. Um von dem piezoelektrischen Vibrator erzeugte Schwingungen auf das Trommelfell zu übertragen, werden gemäß der EP-A-0 340 594 Amboss und Hammer entfernt und das frei schwingende Ende des ansonsten ortsfest festgelegten Vibrators mit dem Steigbügel verbunden.

Aus dem Stand der Technik ist ersichtlich, daß die Erstellung eines vollständig implantierbaren Cochlea Implantates wesentlich auf der technisch und klinisch sinnvollen Realisierung eines adäquaten Mikrofones beruht, das heute aus den dargelegten Gründen nicht zur Verfügung steht

Es ist eine Aufgabe der vorliegenden Erfindung, eine vollständig implantierbare Hörhilfe mit elektrischer Stimulation des Gehörs zu schaffen, die langzeitstabil implantiert werden kann und mit der die oben beschriebenen Probleme bekannter Hörhilfen überwunden werden.

Zur Lösung dieser Aufgabe beschreibt die vorliegende Erfindung eine neue Variante einer vollständig implantierbaren Hörhilfe mit einem implantierbaren Mikrofon, das auf dem von Suzuki und Yanigahara beschriebenen Prinzip eines hermetisch dichten Membrangehäuses beruht, jedoch durch eine prinzipiell abgewandelte Gehäusegeometrie eine deutliche Reduzierung der Außenabmessungen unter Beibehaltung bzw. Verbesserung der funktionalen Betriebsparameter ermöglicht, wobei diese Geometrie den gegebenen, natürlichen anatomischen Bedingungen des Implantationsortes im Bereich der Mastoidhöhle und der hinteren Gehörgangswand angepaßt ist.

Insbesondere wird erfindungsgemäß eine vollständig implantierbare Hörhilfe zur elektrischen Anregung des Gehörs mit einer über ein externes Ladegerät transkutan nachladbaren Akkumulatoranordnung zur elektrischen Energieversorgung, einer über eine externe drahtlose Fernbedienung ansteuerbaren Elektronikeinheit zur audiologischen Signalverarbeitung und Überwachung und Steuerung der internen Energieversorgung, einer Reizelektrodenanordnung zur elektrischen Stimulation des Gehörs und einem implantierbaren Mikrofon mit einer in einem Mikrofongehäuse allseitig hermetisch dicht untergebrachten Mikrofonkapsel und einer elektrischen Durchführungsanordnung zum Durchführen mindestens eines elektrischen Anschlusses von dem Innenraum des Mikrofongehäuses zu dessen Außenseite geschaffen, bei welcher das Mikrofongehäuse mindestens zwei Schenkel aufweist, deren Achsen in einem Winkel mit Bezug aufeinander ausgerichtet sind, wobei der eine Schenkel die Mikrofonkapsel aufnimmt und mit einer Schalleintrittsmembran versehen ist, und wobei der andere Schenkel gegenüber der Ebene der Schalleintrittsmembran zurückversetzt ist und die elektrische Durchführungsanordnung in einem Bereich aufnimmt, der außerhalb einer zur Schalleintrittsmembran senkrechten Projektion der Mikrophonkapsel liegt.

Unter audiologischen Gesichtspunkten ist als optimaler Schallaufnahmeort für das implantierbare Mikrofon einer vollständig implantierbaren Hörhilfe mit elektrischer Stimulation des Gehörs der tiefliegende Bereich des Gehörgangs anzusehen, da dort nahe dem biologisch natürlichen Ort des Trommelfells die akustischen Eigenschaften des Außenohres und des Gehörgangs zur richtungsabhängigen Ausfilterung von Störsignalen ausgenützt werden können. Die vorliegend erläuterte Gestaltung des Mikrofongehäuses gestattet es, den mit der Schalleintrittsmembran versehenen Gehäuseteil bezüglich der geometrischen Abmessungen so weit zu minimieren, daß ein Durchmesser der schallaufnehmenden Membran kleiner 5,0 mm erreicht wird. Detaillierte klinische Untersuchungen haben gezeigt, daß mit diesem Membrandurchmesser noch eine nahezu plane Einpassung an den annähernd kreisförmigen Querschnitt des Gehörgangs im tieferliegenden knöchernen Bereich möglich ist. Dieser minimierte Durchmesser wird bestimmt von den Abmessungen des kleinsten heute verfügbaren Miniatur-Elektret-Mikrofons. Eine interne Plazierung der zusätzlich notwendigen elektronischen Bauelemente sowie der elektrischen Durchführungen zum Anschluß des Moduls neben der Mikrofonkapsel wie in der Ausführungsform von Suzuki und Yanigahara ist dabei nicht möglich. Das Mikrofongehäuse muß nach Mastoidektomie vom Mastoid aus in die in die hintere Gehörgangswand eingebrachte Bohrung geführt und dort so plaziert werden, daß ein mechanisch sicherer Kontakt der gesamten Membranoberfläche mit der über der eingebrachten Bohrung liegenden Gehörgangshaut erzielt wird. Der mit der Schalleintrittsmembran versehene, vorzugsweise zylinderförmige Bereich des Mikrofongehäuses hat daher zweckmäßig mindestens eine Länge, die dem statistischen Maximum der Dicke der hinteren Gehörgangswand entspricht. Als für das neu zu konstruierende Mikrofongehäuse wesentlich geometrie- und volumenbestimmend hat sich der Abstand des Sinus Sigmoideus, dessen Außenkonturen operativ aus klinischen Gründen nicht verändert werden dürfen, zu der hinteren Gehörgangswand herausgestellt. Dieser in manchen individuellen anatomischen Verhältnissen sehr kleine Abstand läßt konstruktiv eine gesamte axiale Anordnung von interner Mikrofonkapsel, elektronischen Bauelementen und der notwendigen hermetischen Signaldurchführung für den elektrischen Anschluß des Mikrofons nicht zu.

Daher ist erfindungsgemäß die grundlegende Geometrie des Mikrofongehäuses so gewählt, daß dieses Gehäuse prinzipiell aus zwei Schenkeln besteht, deren Achsen in einem Winkel zueinander angeordnet sind, wobei der eine Schenkel, der vorzugsweise zylinderförmig ausgeführt ist, die Mikrofonkapsel sowie die schallaufnehmende, vorzugsweise kreisförmige Membran enthält und der andere Schenkel die elektrische Durchführungsanordnung und vorzugsweise elektronische Bauelemente aufnimmt, wie im folgenden näher erläutert wird.

Weitere bevorzugte Ausführungsformen der Erfindung ergeben sich aus den Unteransprüchen.

Insbesondere ist aus den bereits geschilderten Gründen die Geometrie des Mikrofongehäuses bevorzugt so gewählt, daß bei Implantation des Mikrofons in der Mastoidhöhle der Schenkel, der die Schalleintrittsmembran enthält, vom Mastoid aus in eine artifizielle Bohrung in der hinteren, knöchernen Gehörgangswand hineinragt und die Schalleintrittsmembran die Haut der Gehörgangswand berührt, und daß der Schenkel des Mikrofongehäuses, der die elektrische Durchführungsanordnung enthält, im Bereich der Mastoidspitze plaziert ist. Eine über eine vollständige Mastoidektomie hinausgehende Erweiterung der Mastoidhöhle ist hierfür nicht erforderlich.

Da die operative Plazierung des beschriebenen Mikrofonmoduls so erfolgt, daß das Ende des Gehäuseschenkels, der die Durchführungsanordnung enthält, in Richtung der Mastoidspitze weist, ist es zweckmäßig, wenn eine an die Durchführungsanordnung angeschlossene elektrische Leitung im Anschlußbereich in einem rechten Winkel zu dem Gehäuseschenkel steht, der die elektrische Durchführungsanordnung enthält. Dadurch wird ein scharfer Knick bzw. ein sehr kleiner Biegeradius der Leitung vermieden, der andernfalls durch die Anatomie der eröffneten Mastoidhöhle im Bereich der Mastoidspitze vorgegeben wäre, und der Leitungsanschluß an das Mikrofonmodul ist somit bezüglich mechanischer Dauerbeanspruchungen entlastet.

Die elektrische Durchführungsanordnung kann mehrpolig ausgeführt sein. Bei zweipoliger Ausführung ist die Anordnung vorzugsweise so getroffen, daß sie unter Verwendung des elektrischen Prinzips der Phantomspeisung arbeitet, d.h. daß ein Pol gemeinsam das Nutzsignalpotential und das energieversorgende Gleichspannungspotential zur Energieversorgung des Mikrofons führt. Ist die elektrische Durchführungsanordnung einpolig ausgeführt, so kann dieser eine Pol gemeinsam das Nutzsignalpotential und das energieversorgende Gleichspannungspotential führen, während ein zweiter Pol einer zuführenden Leitung an das Mikrofongehäuse angeschlossen ist, das für diesen Zweck elektrisch leitend ausgeführt ist und durch interne Verbindung mit der Mikrofonkaspel-Beschaltung das Massepotential führt.

Unabhängig von der Realisierung der internen Schallwandlung in ein elektrisches Signal sind die zum elektrischen Anschluß der internen Mikrofonkapsel nach dem Prinzip der Phantomspeisung benötigten elektronische Bauelemente und Komponenten vorzugsweise ebenfalls in dem Mikrofongehäuse, insbesondere in dem Gehäuse-Schenkel, der die elektrische Durchführungsanordnung aufnimmt, untergebracht. Ferner können in dem Mikrofongehäuse elektronische Bauelemente untergebracht sein, die eine elektrische Impedanzwandlung und/oder eine elektrische Signalverstärkung und/oder Schutzmaßnahmen gegen elektrische, magnetische und/oder elektromagnetische Umwelteinflüsse ermöglichen. Diese letztgenannten Maßnahmen schließen in der Regel Hochfrequenzkondensatoren und Varistoren ein, um einerseits das Mikrofonmodul selbst gegen Zerstörung aufgrund energiereicher elektromagnetischer Einwirkung zu schützen, und um andererseits kapazitive und induktive Einstreuungen in den Signalpfad zu minimieren bzw. zu unterdrücken, die zu Störungen in der weiteren Audiosignalverarbeitung des an das Mikrofonmodul angeschlossenen Implantatsystems führen können.

Das Mikrofongehäuse einschließlich der schallaufnehmenden Membran und der elektrischen Durchführungseinrichtung ist vorzugsweise mit biokompatiblen Metallen, beispielhaft aus Reintitan oder biokompatiblen Titan-Legierungen, ausgeführt und allseitig hermetisch gasdicht verschlossen.

Das Mikrofongehäuse enthält vorzugsweise eine interne Mikrofonkapsel, die nach dem elektrodynamischen, elektromagnetischen, dielektrischen und vorzugsweise nach dem Elektret-Prinzip arbeitet. Insbesondere kann es sich um eine Elektret-Miniaturausführung mit integriertem Feldeffekt-Transistor zur elektrischen Impedanzwandlung handeln. Dieses Mikrofon kann auch entsprechend dem Kondensator-Mikrofon-Prinzip in NF- bzw. HF-Schaltung ausgeführt sein. Insbesondere kann die Mikrofonfunktion durch ein Halbleiter-Mikrofon in Siliziumtechnologie realisiert sein, das mit Methodiken der Mikrosystemtechnik aufgebaut ist.

Um die elektrische Anschlußleitung weitgehend unempfindlich gegenüber elektromagnetischen Umwelteinflüssen (EMV) machen, ist die elektrische Anschlußleitung, die zweioder mehrpolig ausgeführt sein kann und das Mikrofonmodul mit dem Hörhilfe-Implantatsystem verbindet, vorzugsweise als Twisted-Pair- oder Koaxial-Leitung ausgeführt.

In weiterer Ausgestaltung der Erfindung können das Implantat eine Telemetrieanordnung und das externe Ladegerät eine Kommunikationseinrichtung aufweisen, so daß die Kommunikationseinrichtung über die Telemetrieanordnung Informationen über den energetischen Zustand der implantatseitigen Akkumulatoreinheit liefert kann.

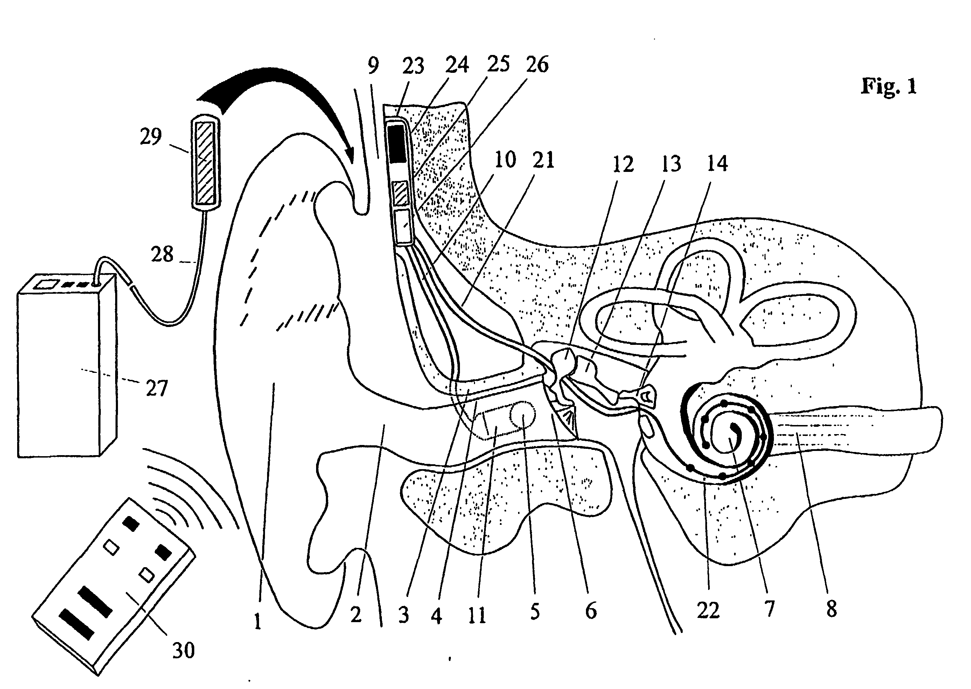

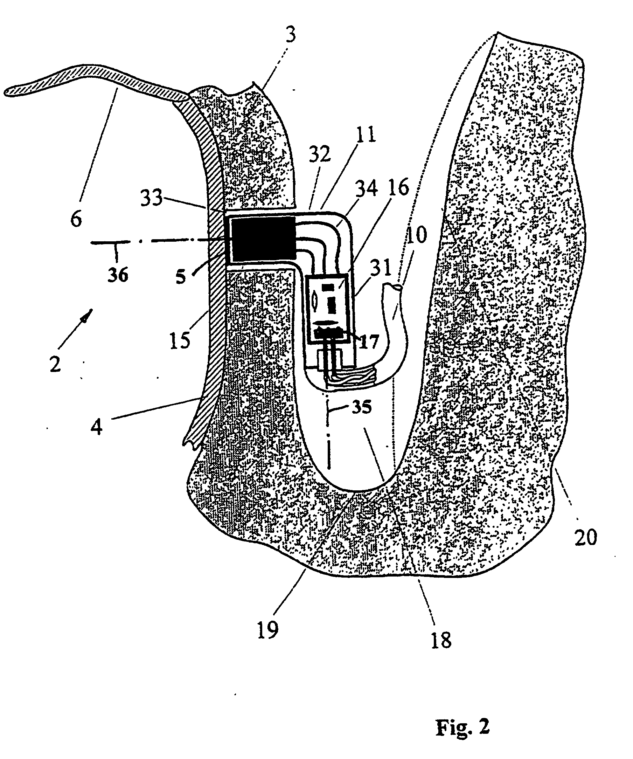

Die beschriebene, erfindungsgemäße Ausführung der vollständig implantierbaren Hörhilfe mit elektrischer Stimulation des Gehörs ist den Abbildungen **Fig. 1** und **Fig. 2** dargestellt Es zeigen:
- **Fig. 1:**: Eine schematische Darstellung des gesamten Hörhilfe-Systems, und
- **Fig. 2:**: eine Aufsicht in die Mastoidhöhle mit implantiertem Mikrofonmodul.

**Fig. 1** zeigt einen Querschnitt durch das menschliche Gehör mit dem Außenohr **1**, dem Gehörgang **2,** der knöchernen Gehörgangswand **3,** der Haut des Gehörgangs **4,** dem Trommelfell **6,** der Gehörknöchelchenkette bestehend aus Hammer **12,** Amboß **13** und Steigbügel **14,** dem Innenohr (Cochlea) **7** und dem Hörnerv **8**. Ein im Bereich der knöchernen hinteren Gehörgangswand vom Mastoid **9** aus eingebrachtes, ein Mikrofongehäuse **11** aufweisendes Mikrofonmodul ist etwa maßstabsgetreu im implantierten Zustand eingezeichnet, wobei die Gehörgangswandhaut **4** eine Membran **5** berührt, welche einen Teil des Mikrofongehäuses **11** bildet. Ein eine elektrische Durchführungsanordnung **17** mit angeschlossener Leitung **10** beinhaltender erster Schenkel **31** des Mikrofongehäuses **11,** dessen Achse in **Fig. 2** schematisch bei **35** angedeutet ist, weist im implantierten Zustand etwa in Richtung der Mastoidspitze. Die elektrische Durchführungsanordnung **17** befindet sich in einem Bereich des Schenkels **31,** der außerhalb einer zu der Schalleintrittsmembran **5** senkrechten Projektion der Mikrofonkapsel **15** liegt.

Die elektrische Leitung **10** des Mikrofons führt zu einem Hauptelektronikmodul **23,** das in einer Ausfräsung im knöchemen Bereich des Mastoids **9** untergebracht ist. Das Hauptelektronikmodul **23** enthält eine aus einem oder mehreren wiederaufladbaren Akkumulatoren bestehende Akkumulatoranordnung **25**, eine Empfangseinrichtung **24** für den Empfang der transkutan und induktiv zugeführten Energie zum Nachladen der Akkumulatoranordnung **25**, eine Elektronikeinheit **26** zur Audiosignalverarbeitung und zur Steuerung und Regelung der internen Energieversorgung, wobei die Elektronikeinheit **26** eine Telemetrieeinrichtung zur Datenkommunikation mit der Außenwelt einschließt. Von dem Hauptelektronikmodul **23** führt eine Leitung **21** zu der hier mehrkanalig dargestellten Reizelektrodenanordnung **22**, die in die basale Windung des Innenohrs (Cochlea) **7** eingeführt ist

Das Implantat wird zum Gesamtsystem ergänzt durch ein portabel ausgeführtes Ladegerät **27** und eine ebenfalls portable, drahtlose Fernbedienung **30.** Zum induktiven Nachladen der implantatseitigen Akkumulatoranordnung **25** wird temporär eine über eine Leitung **28** an das Ladegerät **27** angeschlossene Sendespule **29** hinter das Außenohr **1** über die implantatseitige Empfangseinrichtung **24** gebracht, wie dies in **Fig. 1** durch einen Pfeil angedeutet ist. Mit Hilfe der Fernbedienung **30** lassen sich die Betriebsparameter des Systems vom Patienten verändern und den alltäglichen akustischen Umgebungsbedingungen anpassen. Die audiologische Grundanpassung des Implantates erfolgt ebenfalls transkutan durch ein Programmiersystem, das mit der implantatseitigen Telemetrieeinrichtung kommuniziert.

In **Fig. 2** ist das Mikrofongehäuse **11** schematisch im implantierten Zustand im Querschnitt als Aufsicht auf die eröffnete Mastoidhöhle **18** nach Mastoidektomie dargestellt. Das Mikrofongehäuse **11** ist mit einem eine Mikrofonkapsel **15** aufnehmenden zweiten Gehäuseschenkel **32** so in eine entsprechende artifizielle Bohrung **33** in der knöchernen Gehörgangswand **3** eingeführt, daß die Haut **4** des Gehörgangs die kreisförmige Membran **5** mechanisch sicher und auf der ganzen Kreisfläche der Membran berührt. Der Gehäuseschenkel **32** hat eine Achse **36 (Fig. 2),** die in der veranschaulichten Ausführungsform im wesentlichen in einem rechten Winkel zu der Achse **35** des Gehäuseschenkels **31** steht. Der Bereich der Gehörgangswandhaut, der die Membran berührt, stellt zusammen mit der Membran ein mechanisch schwingfähiges Gebilde dar, das durch eine in den äußeren Gehörgang **2** mit dem Trommelfell **6** einfallende Schallwelle in mechanische Schwingungen versetzt wird, die sich als akustische Druckschwankungen im hermetisch abgeschlossenen inneren Volumen des Mikrofongehäuses **11** abbilden. Diese Druckschwankungen werden durch die interne Mikrofonkapsel **15** in ein elektrisches Signal umgewandelt, das einer Signalverarbeitungseinheit **16** über interne elektrische Leitungen **34** zugeführt wird.

Die Signalverarbeitungseinheit **16** kann Komponenten zur elektrischen Phantomspeisung des Systems, aktiv verstärkende Elemente, impedanzwandelnde Bauelemente und Komponenten zur Unterdrückung bzw. Dämpfung von elektrischen, magnetischen und/oder elektromagnetischen Umwelteinflüssen beinhalten. Die Signalverarbeitungseinheit **16** ist ausgangsseitig elektrisch an die Durchführungsanordnung **17** angeschlossen, die hermetisch dicht in das Mikrofongehäuse **11** eingebracht ist. Die Signalverarbeitungseinheit **16** ist zusammen mit der Durchführungsanordnung **17** in dem Schenkel **31** des Mikrofongehäuses **11** untergebracht, der unter einem Winkel, hier vorzugsweise als rechter Winkel dargestellt, zu dem Schenkel **32** des Mikrofongehäuses steht, der mit der schallaufnehmenden Membran **5** versehen ist und die interne Mikrofonkapsel **15** enthält. Die elektrische Leitung **10** ist vorzugsweise im rechten Winkel so an die elektrische Durchführungsanordnung **17** angeschlossen, daß die Leitung **10** knickfrei und unter Vermeidung sehr kleiner Biegeradien aus der Mastoidspitze **19** herausgeführt werden kann. Die elektrische Durchführungsanordnung **17** und die Leitung **10** sind hier für den vorzugsweisen Fall einer energetischen Versorgung des Mikrofonmoduls über eine Phantomspeisung, bei der die Nutzsignal-Wechselspannung und die versorgende Gleichspannung gemeinsam über einen Pol und das Massesignal über einen zweiten Pol geführt werden, zweipolig ausgeführt. In diesem Fall ist die Leitungsanordnung der elektrischen Implantatleitung **10** vorzugsweise nach dem Twisted-Pair-Prinzip ausgeführt, um elektromagnetische Umwelteinflüsse auf die Implantatleitung **10** zu minimieren.

Aus **Fig. 2** sind die beengten Raumverhältnisse in der eröffneten Mastoidhöhle ersichtlich, die wesentlich durch den Abstand zwischen hinterer Gehörgangswand **3** und dem knöchernen Wall des Sinus Sigmoideus **20**, der eine wichtige venöse Versorgung enthält und daher chirurgisch nicht abgetragen werden kann, gegeben sind. Erst die Unterbringung der beschriebenen Komponenten in einem zweischenkeligen Gehäuse erlaubt die Wahl des dargestellten Implantationsortes. Eine axiale Anordnung aller Mikrofon-Modul-Komponenten einschließlich des elektrischen Leitungsanschlusses ist nicht möglich. Weiterhin ist aus **Fig. 2** ersichtlich, daß der die Durchführungsanordnung **17** enthaltende Schenkel **31** des Mikrofongehäuses **11** gegenüber der Ebene der kreisförmigen Gehäusemembran **5** mindestens um die Dicke der knöchernen Gehörgangswand **3** zurückgesetzt sein muß, um ein so tiefes Einführen des Gehäuses **11** in die artifizielle Bohrung **33** in der Gehörgangswand zu gewährleisten, daß die Membran **5** des Mikrofongehäuses die Haut **4** der Gehörgangswand sicher in allen Membranbereichen berührt.

## Patentansprüche

1. Vollständig implantierbare Hörhilfe zur elektrischen Anregung des Gehörs mit einer über ein externes Ladegerät (27) transkutan nachladbaren Akkumulatoranordnung (25) zur elektrischen Energieversorgung, einer über eine externe drahtlose Fernbedienung (30) ansteuerbaren Elektronikeinheit (26) zur audiologischen Signalverarbeitung und Überwachung und Steuerung der internen Energieversorgung, einer Reizelektrodenanordnung (22) zur elektrischen Stimulation des Gehörs (7) und einem implantierbaren Mikrofon mit einer in einem Mikrofongehäuse (11) allseitig hermetisch dicht untergebrachten Mikrofonkapsel (15) und einer elektrischen Durchführungsanordnung (17) zum Durchführen mindestens eines elektrischen Anschlusses von dem Innenraum des Mikrofongehäuses zu dessen Außenseite, wobei das Mikrofongehäuse (11) mindestens zwei Schenkel (31, 32) aufweist, deren Achsen (35, 36) in einem Winkel mit Bezug aufeinander ausgerichtet sind, wobei der eine Schenkel (32) die Mikrofonkapsel (15) aufnimmt und mit einer Schalleintrittsmembran (5) versehen ist, und wobei der andere Schenkel (31) gegenüber der Ebene der Schalleintrittsmembran zurückversetzt ist und die elektrische Durchführungsanordnung (17) in einem Bereich aufnimmt, der außerhalb einer zur Schalleintrittsmembran senkrechten Projektion der Mikrophonkapsel liegt.

2. Vollständig implantierbare Hörhilfe nach Anspruch 1, **dadurch gekennzeichnet, daß** die Geometrie des Mikrofongehäuses (11) so gewählt ist, daß bei Implantation des Mikrofons in der Mastoidhöhle (18) der Schenkel (32), der die Schalleintrittsmembran (5) enthält, vom Mastoid (9) aus in eine artifizielle Bohrung (33) in der hinteren, knöchernen Gehörgangswand (3) hineinragt und die Schalleintrittsmembran die Haut (4) der Gehörgangswand berührt, und daß der Schenkel (31) des Mikrofongehäuses, der die elektrische Durchführungsanordnung (17) enthält, im Bereich der Mastoidspitze (19) plaziert ist.

3. Vollständig implantierbare Hörhilfe nach einem der vorhergehenden Ansprüche, **dadurch gekennzeichnet, daß** der von den Achsen (35, 36) der beiden Schenkel (31, 32) des Mikrofongehäuses (11) gebildete Winkel näherungsweise ein rechter Winkel ist

4. Vollständig implantierbare Hörhilfe nach einem der vorhergehenden Ansprüche, **dadurch gekennzeichnet, daß** an die Durchführungsanordnung (17) des Mikrofons eine elektrische Leitung (10) angeschlossen ist, die im Anschlußbereich in einem rechten Winkel zu dem Gehäuseschenkel (31) steht, der die elektrische Durchführungsanordnung enthält.

5. Vollständig implantierbare Hörhilfe nach einem der vorhergehenden Ansprüche, **dadurch gekennzeichnet, daß** die elektrische Durchführungsanordnung (17) des Mikrofons mehrpolig ausgeführt ist.

6. Vollständig implantierbare Hörhilfe nach einem der vorhergehenden Ansprüche, **dadurch gekennzeichnet, daß** die elektrische Durchführungsanordnung (17) des Mikrofons zweipolig unter Verwendung des elektrischen Prinzips der Phantomspeisung ausgeführt ist.

7. Vollständig implantierbare Hörhilfe nach einem der Ansprüche 1 bis 4, **dadurch gekennzeichnet, daß** die elektrische Durchführungsanordnung (17) einpolig ausgeführt ist und dieser eine Pol gemeinsam das Nutzsignalpotential und das energieversorgende Gleichspannungspotential führt, daß das Mikrofongehäuse (11) elektrisch leitend ausgeführt ist, und daß ein zweiter Pol einer zuführenden Leitung (10) angeschlossen ist und das Massepotential über das Mikrofongehäuse geführt ist.

8. Vollständig implantierbare Hörhilfe nach einem der vorhergehenden Ansprüche, **dadurch gekennzeichnet, daß** in dem Mikrofongehäuse (11) ferner elektronische Bauelemente (16) untergebracht sind, die einen elektrischen Anschluß der internen Mikrofonkapsel (15) nach dem Prinzip der Phantomspeisung ermöglichen.

9. Vollständig implantierbare Hörhilfe nach einem der vorhergehenden Ansprüche, **dadurch gekennzeichnet, daß** in dem Mikrofongehäuse (11) ferner elektronische Bauelemente (16) untergebracht sind, die eine Minimierung elektrischer, magnetischer und/oder elektromagnetischer Umwelteinflüsse gewährleisten.

10. Vollständig implantierbare Hörhilfe nach einem der vorhergehenden Ansprüche, **dadurch gekennzeichnet, daß** in dem Mikrofongehäuse (11) ferner elektronische Bauelemente (16) untergebracht sind, die eine elektrische Verstärkung und/oder eine Impedanzwandlung des Mikrofonsignals realisieren.

11. Vollständig implantierbare Hörhilfe nach einem der vorhergehenden Ansprüche, **dadurch gekennzeichnet, daß** das Mikrofongehäuse (11) einschließlich der Schalleintrittsmembran (5) in Reintitan oder biokompatiblen Titan-Legierungen ausgeführt ist.

12. Vollständig implantierbare Hörhilfe nach einem der vorhergehenden Ansprüche, **dadurch gekennzeichnet, daß** die Mikrofonkapsel (15) zum Arbeiten nach dem elektrodynamischen, elektromagnetischen, dielektrischen, piezoelektrischen oder vorzugsweise nach dem Elektret-Prinzip ausgelegt ist.

13. Vollständig implantierbare Hörhilfe nach einem der vorhergehenden Ansprüche, **dadurch gekennzeichnet, daß** das Mikrofongehäuse (11) ein internes Halbleiter-Mikrofon (15) enthält, das nach Methoden der Mikrosystemtechnik gefertigt ist.

14. Vollständig implantierbare Hörhilfe nach einem der vorhergehenden Ansprüche, **dadurch gekennzeichnet, daß** die externe, elektrische Anschlußleitung (10) als Twisted-Pair- oder Koaxial-Leitung ausgeführt ist.

15. Vollständig implantierbare Hörhilfe nach einem der vorhergehenden Ansprüche, **dadurch gekennzeichnet, daß** die implantatseitige Akkumulatoranordnung (25) aus einer oder mehreren Nickel-Cadmium-, Nickel-Metall-Hydrid-, Lithium- oder Lithium-Ionen-Zellen besteht.

16. Vollständig implantierbare Hörhilfe nach einem der vorhergehenden Ansprüche, **dadurch gekennzeichnet, daß** implantatseitig eine Telemetrieanordnung vorgesehen ist und daß das externe Ladegerät (27) eine Kommunikationseinrichtung beinhaltet, die über die Telemetrieanordnung Informationen über den energetischen Zustand der implantatseitigen Akkumulatoreinheit (25) liefert.

17. Vollständig implantierbare Hörhilfe nach einem der vorhergehenden Ansprüche, **dadurch gekennzeichnet, daß** die Fernbedienung (30) für eine Übertragung von Steuerungsdaten an die implantatseitige Elektronik (26) auf induktivem Weg oder über eine Hochfrequenzstrecke ausgelegt ist.

## Claims

1. Fully implantable hearing aid for electric stimulation of the hearing, having a battery arrangement (25) for supplying electrical energy, transcutaneously rechargeable via an external charging device (27), an electronic unit (26), capable of being triggered via an external wireless remote control (30), for processing audiological signals and monitoring and controlling the internal power supply, a stimulating electrode device (22) for electrically stimulating the hearing (7), and an implantable microphone having a microphone capsule (15), which is housed in the microphone housing (11) hermetically sealed all round, and an electric lead arrangement (17) for leading through at least one electrical connection from the interior of the microphone housing to its exterior, wherein the microphone housing (11) has at least two arms (31, 32), whose axes (35, 36) are aligned at an angle to one another, wherein the one arm (32) receives the microphone capsule (15) and is provided with a sound entry membrane (5), and wherein the other arm (31) is offset from the plane of the sound entry membrane and receives the electric lead arrangement (17) in a region lying outside a projection of the microphone capsule perpendicular to the sound entry membrane.

2. Fully implantable hearing aid according to claim 1, **characterised in that** the geometry of the microphone housing (11) is such that when the microphone is being implanted into the mastoid cavity (18) the arm (32), which contains the sound entry membrane (5) projects from the mastoid (9) into an artificial bore (33) in the rear, bony wall (3) of the auditory canal and the sound entry membrane touches the skin (4) of the wall of the auditory canal, and **in that** the arm (31) of the microphone housing containing the electric lead arrangement (17) is placed in the region of the mastoid tip (19).

3. Fully implantable hearing aid according to one of the preceding claims, **characterised in that** the angle formed by the axes (35, 36) of the two arms (31, 32) of the microphone housing (11) is approximately a right angle.

4. Fully implantable hearing aid according to one of the preceding claims, **characterised in that** an electric line (10) is connected to the lead arrangement (17) of the microphone and at the joining region is at a right angle to the housing arm (31) carrying the electric lead arrangement.

5. Fully implantable hearing aid according to one of the preceding claims, **characterised in that** the electric lead arrangement (17) of the microphone is multi-pole.

6. Fully implantable hearing aid according to one of the preceding claims, **characterised in that** the electric lead arrangement (17) of the microphone is bipolar, using the electrical principle of phantom feed.

7. Fully implantable hearing aid according to one of the preceding claims, **characterised in that** the electric lead arrangement (17) is single-pole and this one pole conducts together the wanted signal potential and the power-supplying direct voltage potential, **in that** the microphone housing (11) is electrically conductive, and **in that** a second pole is connected to a supplying line (10) and the zero potential is conducted via the microphone housing.

8. Fully implantable hearing aid according to one of the preceding claims, **characterised in that** also housed in the microphone housing (11) are electronic components (16) which permit electrical connection of the internal microphone capsule (15) according to the principle of phantom feed.

9. Fully implantable hearing aid according to one of the preceding claims, **characterised in that** also housed in the microphone housing (11) are electronic components (16) which minimise electrical, magnetic and/or electromagnetic environmental effects.

10. Fully implantable hearing aid according to one of the preceding claims, **characterised in that** also housed in the microphone housing (11) are electronic components (16) which realise electrical amplification and/or impedance conversion of the microphone signal.

11. Fully implantable hearing aid according to one of the preceding claims, **characterised in that** the microphone housing (11) including the sound entry membrane (5) is formed of pure titanium or biocompatible titanium alloy.

12. Fully implantable hearing aid according to one of the preceding claims, **characterised in that** the microphone capsule (15) is formed, for operation, according to the electrodynamic, electromagnetic, dielectric, piezoelectric or preferably according to the electret principle.

13. Fully implantable hearing aid according to one of the preceding claims, **characterised in that** the microphone housing (11) contains an internal semiconductor microphone (15) which is manufactured according to methods of micro-system technology.

14. Fully implantable hearing aid according to one of the preceding claims, **characterised in that** the external, electrical connection line (10) is formed as a twisted pair or coaxial cable.

15. Fully implantable hearing aid according to one of the preceding claims, **characterised in that** the battery arrangement (25) on the implant side consists of one or more nickelcadmium, nickel-metal hydride, lithium or lithium ion cells.

16. Fully implantable hearing aid according to one of the preceding claims, **characterised in that** on the implant side a telemetry arrangement is provided, and **in that** the external charging device (27) contains a communication device, which supplies via the telemetry arrangement data about the power level of the implant-side battery pack (25).

17. Fully implantable hearing aid according to one of the preceding claims, **characterised in that** the remote control (30) is wired inductively or via a high-frequency section in order to transmit control data to the implant-side electronics (26).

## Revendications

1. Prothèse auditive pouvant être entièrement implantée destinée à l'excitation électrique de l'ouïe, comportant un dispositif d'accumulateurs (25) pour l'alimentation en énergie électrique pouvant être rechargé par voie transcutanée par l'intermédiaire d'un chargeur externe (27), une unité électronique (26) pouvant être commandée par l'intermédiaire d'une télécommande externe (30) sans fil pour le traitement audiologique des signaux, la surveillance et la commande de l'alimentation interne en énergie, un dispositif d'électrodes d'excitation (22) pour la stimulation électrique de l'ouïe (7), et un microphone pouvant être implanté avec une capsule microphonique (15) logée dans un boîtier (11) de microphone en étant hermétiquement étanche de tous côtés, et un dispositif de traversée électrique (17) pour le passage d'au moins un raccordement électrique de l'espace intérieur du boîtier du microphone vers la face extérieure de ce dernier, le boîtier (11) du microphone comportant au moins deux branches (31, 32) dont les axes (35, 36) sont orientés suivant un angle l'un par rapport à l'autre, l'une des branches (32) recevant la capsule microphonique (15) et étant munie d'une membrane d'entrée acoustique (5), et l'autre branche (31) étant décalée vers l'arrière par rapport au plan de la membrane d'entrée acoustique, et recevant le dispositif de traversée électrique (17) dans une zone qui est située en-dehors d'une projection de la capsule microphonique perpendiculaire à la membrane d'entrée acoustique.

2. Prothèse auditive pouvant être entièrement implantée selon la revendication 1, **caractérisée en ce que** la géométrie du boîtier (11) du microphone est choisie de telle sorte que, lors de l'implantation du microphone dans la cavité (18) de la mastoïde, la branche (32) contenant la membrane d'entrée acoustique (5) pénètre à partir de la mastoïde (9) dans un perçage artificiel (33) pratiqué dans la paroi osseuse arrière (3) du conduit auditif, et que la membrane d'entrée acoustique soit en contact avec la peau (4) de la paroi du conduit auditif, et **en ce que** la branche (31) du boîtier du microphone contenant le dispositif de traversée électrique (17) est placée dans la zone de l'apophyse (19) de la mastoïde.

3. Prothèse auditive pouvant être entièrement implantée selon l'une des revendications précédentes, **caractérisée en ce que** l'angle formé par les axes (35, 36) des deux branches (31, 32) du boîtier (11) du microphone est sensiblement un angle droit.

4. Prothèse auditive pouvant être entièrement implantée selon l'une quelconque des revendications précédentes, **caractérisée en ce qu'**une ligne électrique (10) est raccordée au dispositif de traversée (17) du microphone, laquelle est située dans la zone de raccordement à angle droit par rapport à la branche (31) du boîtier contenant le dispositif de traversée électrique.

5. Prothèse auditive pouvant être entièrement implantée selon l'une quelconque des revendications précédentes, **caractérisée en ce que** le dispositif de traversée électrique (17) du microphone est du type multipolaire.

6. Prothèse auditive pouvant être entièrement implantée selon l'une quelconque des revendications précédentes, **caractérisée en ce que** le dispositif de traversée électrique (17) du microphone est du type bipolaire utilisant le principe électrique de l'alimentation fantôme.

7. Prothèse auditive pouvant être entièrement implantée selon l'une quelconque des revendications 1 à 4, **caractérisée en ce que** le dispositif de traversée électrique (17) est du type unipolaire, et **en ce que** ce seul pôle conduit en commun le potentiel du signal utile et le potentiel de tension continue d'alimentation en énergie, **en ce que** le boîtier (11) du microphone est réalisé en étant électriquement conducteur, et **en ce qu'**un deuxième pôle d'une ligne d'alimentation (10) y est raccordé, et **en ce que** le potentiel de masse passe par le boîtier du microphone.

8. Prothèse auditive pouvant être entièrement implantée selon l'une quelconque des revendications précédentes, **caractérisée en ce que** des composants électroniques (16) sont en outre logés dans le boîtier (11) du microphone, qui permettent un raccordement électrique de la capsule microphonique interne (15) selon le principe de l'alimentation fantôme.

9. Prothèse auditive pouvant être entièrement implantée selon l'une quelconque des revendications précédentes, **caractérisée en ce que** des composants électroniques (16) sont en outre logés dans le boîtier (11) du microphone, qui minimisent des influences électriques, magnétiques et/ou électromagnétiques provenant de l'environnement.

10. Prothèse auditive pouvant être entièrement implantée selon l'une quelconque des revendications précédentes, **caractérisée en ce que** des composants électroniques (16) sont en outre logés dans le boîtier (11) de microphone, qui réalisent une amplification électrique et/ou une transformation d'impédance du signal du microphone.

11. Prothèse auditive pouvant être entièrement implantée selon l'une quelconque des revendications précédentes, **caractérisée en ce que** le boîtier (11) du microphone, y compris la membrane d'entrée acoustique (5), sont réalisés en titane pur ou en alliages de titane biologiquement compatibles.

12. Prothèse auditive pouvant être entièrement implantée selon l'une quelconque des revendications précédentes, **caractérisée en ce que** la capsule microphonique (15) est conçue pour un fonctionnement selon le principe électrodynamique, électromagnétique, diélectrique, piézoélectrique ou de préférence selon le principe de l'électret.

13. Prothèse auditive pouvant être entièrement implantée selon l'une quelconque des revendications précédentes, **caractérisée en ce que** le boîtier (11) du microphone contient un microphone interne (15) à semiconducteurs qui est réalisé selon la méthode de la technique des microsystèmes.

14. Prothèse auditive pouvant être entièrement implantée selon l'une quelconque des revendications précédentes, **caractérisée en ce que** la ligne de raccordement électrique externe (10) est réalisée sous la forme d'une ligne twisted-pair ou coaxiale.

15. Prothèse auditive pouvant être entièrement implantée selon l'une quelconque des revendications précédentes, **caractérisée en ce que** le dispositif d'accumulateurs (25) situé du côté de l'implant est constitué d'un ou de plusieurs éléments au nickel-cadmium, au nickel-métal-hydrure, au lithium ou aux ions de lithium.

16. Prothèse auditive pouvant être entièrement implantée selon l'une quelconque des revendications précédentes, **caractérisée en ce qu'**un dispositif de télémétrie est prévu du côté de l'implant, et **en ce que** le chargeur externe (27) comporte un dispositif de communication qui, par l'intermédiaire du dispositif de télémétrie, fournit des informations sur l'état énergétique du dispositif d'accumulateurs (25) situé du côté de l'implant.

17. Prothèse auditive pouvant être entièrement implantée selon l'une quelconque des revendications précédentes, **caractérisée en ce que** la télécommande (30) est conçue pour une transmission par voie inductive ou par l'intermédiaire d'une liaison à haute fréquence de données de commande à l'électronique (26) située du côté de l'implant.
